Europäisches Patentamt

European Patent Office

Office européen des brevets

(11) Publication number: **0 209 957**
**B1**

# EUROPEAN PATENT SPECIFICATION

(45) Date of publication of patent specification: **24.05.89**

(51) Int. Cl.⁴: **A 61 K 31/195, A 61 K 9/20**

(21) Application number: **86201294.5**

(22) Date of filing: **22.07.86**

(54) Tablets containing S-carboxymethylcysteine.

(30) Priority: **22.07.85 NL 8502092**

(43) Date of publication of application:
**28.01.87 Bulletin 87/05**

(45) Publication of the grant of the patent:
**24.05.89 Bulletin 89/21**

(84) Designated Contracting States:
**AT BE CH DE FR GB IT LI LU NL SE**

(56) References cited:
**EP-A-0 123 668**
**EP-A-0 125 634**
**GB-A- 974 917**

**DICTIONNAIRE VIDAL, 60th edition, 1984, OVP,**
**Paris, FR; p. 1388, "Thio-Théo, comprimes"**

(73) Proprietor: **Cedona Pharmaceuticals B.V.**
**Oudeweg 147**
**NL-2031 CC Haarlem (NL)**

(72) Inventor: **Bron, Jan**
**Koninginneweg 109**
**NL-1075 CK Amsterdam (NL)**

(74) Representative: **Kroesen, Johan Albert Carel**
**et al**
**Octrooibureau Vriesendorp & Gaade Postbus**
**266**
**NL-2501 AW The Hague (NL)**

Courier Press, Leamington Spa, England.

## Description

Said invention relates to a tablet or tablet kernel containng S-carboxymethylcysteine and carrier and adjuvants known in carriers and adjuvants.

Tablets containing S-carboxymethylcysteine are i.a. known from U.S. Patent Specification 3 891 749 (and German patent specification 2 006 486 corresponding thereto). S-carboxymethylcysteine has various activities of which thinning and dissolving phlegm in the bronchia (thus a diminution of the symptoms of bronchitis and pneumonia) is the most important one. (Vide French Patent Specifications 2 398 497, 2 430 945 and 2 506 614 and Belgian Patent Specification 887 922). Beside this it also decreases seborrhoea (vide U.S. Patent Specification 3 671 643 and 3 950 542, British Patent Specification 1 051 870 and Dutch Patent Specification 152 169).

As relatively high amounts of S-carboxymethylcysteine should be administered (generally 2—2.5 g daily, often divided in 3 dosages) tablets with high contents of said substance are desired. Such tablets are hard to prepare. The properties of tablets or tablet kernels with modest content of active principle (up to about 1—2%) are completely determined by the lactose or the mixture of lactose and saccharose of which the tablet substantially consists in most cases, but for tablets and kernels with high contents of active principle the properties of the active principle itself are determining when compressing.

In the above-mentioned U.S. Patent Specification 3 891 749 tablets are mentioned which per 100 mg of S-carboxymethylcysteine contain 100 mg of saccharose, 45 mg of starch and 5 mg of magnesium stearate. It appeared, however, that a granulate having this composition could not be compressed according to the practice of the art into tablets, the mass kept sticking to the dies. Also blends having closely similar compositions should not be processed at all into tablets or kernels. Moreover, it may be expected the formulation described in U.S. Patent Specification 3 891 749, on account of the lack of tablet adjuvants for desintegration of the tablet according to the requirements of the international Pharmacopoeias, that the active principle will be insufficiently available to attain the clinical effect aimed at. This is supported by the fact that nowadays compositions containing S-carboxymethylcysteine are exclusively commercially available as mixture or capsules, having an equivalent biological availability. Consequently, in view of their pharmaceutical and technological possibilities to process high doses of S-carboxymethylcysteine, drinks, suspensions and capsules containing S-carboxymethylcysteine are still the obvious administration forms for high doses of S-carboxymethylcysteine.

Now it was found that said objections are removed by a well realizable tablet or tablet kernel if the adjuvants therein are a blend of 1—5 parts by weight of mannitol, 0.2—2 parts by weight of talcum 0.2—2 parts by weight of colloidal silica, 0.1—1 parts by weight of stearic acid and/or salts thereof such as magnesium, calcium or aluminium stearate, and 0.2—4 parts by weight of starch on 1 part by weight of crospovidone, the S-carboxymethylcysteine content being from 10 to 90% by weight.

Preferably said adjuvants for tablets on tablet kernels are a blend of 1—3 parts by weight of mannitol, 0.2—1 part by weight of talcum, 0.2—1 part by weight of colloidal silica, 0.1—0.5 parts of weight of stearic acid or salt thereof and 0.5—2 parts by weight of starch on 1 part by weight of Crospovidone, and preferably the content of S-carboxymethylcysteine is between 40 and 80% by weight.

The best results are obained with a tablet containing 375 mg of S-carboxymethylcysteine, about 50 mg of mannitol, about 10 mg of talcum, about 10 mg of colloidal silica and about 5 mg of magnesium stearic on about 25 mg of Crospovidone, beside some starch for granulating and bringing the tablet at weight. This corresponds to 2 parts by weight of mannitol, 0.4 parts of talcum, 0.4 parts by weight of colloidal silica, 0.2 parts by weight of stearic acid or a salt thereof and 1.5 parts by weight of starch on 1 part by weight of Crospovidone.

Apart from the talcum, the colloidal silica, the Crospovidone, the starch and the stearic acid or salt thereof, said tablets are completely soluble in water and in gastric and intestinal juices. The tablets quickly fall apart, within the 15 minutes required by the international pharmacopeias. The decomposition time of the composition according to the invention is generally from 0.5 to 5 minutes.

The biological availability of S-carboxylmethylcysteine after administration of tablets containing 375 mg S-carboxymethylcystyeine was compared with those after administration of the same amount of S-carboxymethylcysteine in the form of capsules and mixtures to 9 healthy volunteers. It can be shown that the developed tablet formulation of S-carboxymethylcysteine has the same biological availability as a mixture or a capsule containing S-carboxymethylcysteine. Thus the biological availability of the S-carboxymethylcysteine in the tablets is complete.

All components are known substances, of course they should be of a quality which is acceptable for pharmaceutical compositions. The presence of a slight amount of accidential diluents, such as adsorbed water is acceptable, provided that said casual diluents are pharmaceutically inert.

Crospovidone is a branched polyvinylpyrrolidone, it should meet the specification of the American Pharmacopoea (in the edition of January 1, 1985 on page 554-5). Also the remaining adjuvants should meet the requirements of the Dutch, resp. European or American Pharmacopoea.

The invention also relates to a process for preparing tablets of tablet kernels containing from 10 to 90% by weight of S-carboxymethylcysteine. In said process S-carboxymethylcysteine is

mixed with mannitol, is granulated with a blend of starch and water, is dried, sieved again and after adding the remaining adjuvants is compressed to tablets or tablet kernels. The novel process is characterized in that for granulating and compressing as adjuvants for every part by weight of Crospovidone 1—5 parts by weight of mannitol, 0.2—2 parts by weight of talcum, 0.2—2 parts by weight of colloidal silica, 0.1—1 part by weight of stearic acid and/or a salt thereof such as magnesium, calcium, or aluminiumstearate and 0.2—4 parts by weight of starch are used.

Preferably per 1 part by weight of Crospovidone 1—3 parts by weight of mannitol, 0.2—1 parts by weight of talcum, 0.2—1 part by weight of colloidal silica, 0.1—0.5 part by weight of stearic acid or salt thereof and 0.5—2 parts by weight of starch are used.

## Example

A blend of 375 g of S-carboxymethylcysteine and 50 g of mannitol was moistened with 125 ml of a 10% starch suspension and granulated by a sieve of 8 mm. The moistened mass was dried, sieved again through a sieve of 1 mm, and after mixing it with 26 g Crospovidone, 5.2 g of magnesium stearate, 10.4 g of talcum and 10.4 g of colloidal silica and starch up to 520 g almost 1000 tablets were formed of it. This happened without difficulty and interruption. The dies kept completely clean.

## Claims

1. Tablet or tablet kernel containing S-carboxymethylcysteine and carriers and adjuvants known for tablets, characterized in that the adjuvants in it are a blend of 1—5 parts by weight of mannitol 0.2—2 parts by weight of talcum, 0.2—2 parts by weight of colloidal silica, 0.1—1 part by weight of stearic acid or salt thereof and 0.2—4 parts by weight of starch on 1 part by weight of Crospovidone, the S-carboxymethylcysteine content being from 10 to 90% by weight.

2. Tablet or tablet kernel according to Claim 1, characterized in that the adjuvants in it are a mixture of 1—3 parts by weight of mannitol, 0.2—1 part by weight of talcum, 0.2—1 part by weight of colloidal silica, 0.1—0.5 parts by weight of stearic acid or salt thereof and 0.5—2 parts by weight of starch on 1 part by weight of Crospovidone, the S-carboxymethylcysteine content being from 40 to 80% by weight.

3. Tablet or tablet kernel according to Claim 2, characterized in that the blend of adjuvants in it is a blend of about 2 parts by weight of mannitol, about 0.4 parts by weight of talcum, about 0.4 parts by weight of colloidal silica, about 0.2 parts by weight of stearic acid or a salt thereof and 1.5 parts by weight of starch on 1 part by weight of Crospovidone.

4. Process for preparing tablets or tablet kernels, in which S-carboxymethylcysteine is blended with mannitol and granulated with a blend of starch and water, dried, sieved again and after adding the remaining adjuvants are compressed to tablets or tablet kernels, characterized in that as adjuvants for every part by weight of Crospovidone 1—5 parts by weight of mannitol, 0.2—2 parts by weight of talcum, 0.2—2 parts by weight of colloidal silica, 0.1—2 part by weight of stearic acid or salt thereof and 0.2—4 parts by weight of starch are used, the S-carboxymethylcysteine content of the blend being from 10 to 90% by weight.

5. Process according to Claim 4, characterized in that as adjuvants for every part by weight of Crospovidone 1—3 parts by weight of mannitol, 0.2—1 part by weight of talcum, 0.2—1 part by weight of colloidal silica, 0.1—0.5 parts by weight of stearic acid or a salt thereof and 0.5—2 parts by weight of starch are used.

6. Tablet or tablet kernel obtainable by the process of Claim 4 or 5.

## Patentansprüche

1. Tablette oder Dragéekern, das/der S-Carboxymethylcystein und in Tabletten bekannte Träger und Hilfsstoffe enthält, dadurch gekennzeichnet, dass die Hilfstoffe darin ein Gemisch von 1—5 Gew.Teile Mannitol, 0,2—2 Gew.Teile Talk, 0,2—2 Gew.Teile kolloïdale Kieselsäure, 0,1—1 Gew.Teile Stearinsäure oder dessen Salz und 0,2—4 Gewichtsteile Stärke auf 1 Gew.- Teil Crospovidon sind, wobei der S-Carboxymethylcystein-Gehalt zwischen 10 und 90 Gew.-% liegt.

2. Tablette oder Dragéekern nach Anspruch 1, dadurch gekennzeichnet, dass die Hilfsstoffe darin ein Gemisch von 1—3 Gew.Teile Mannitol, 0,2—1 Gew.Teile Talk, 0,2—1 Gew. Teil kolloïdale Kieselsäure, 0,1—0,5 Gew.Teile Stearinsäure oder dessen Salz und 0,5—2 Gew.Teile Stärke auf 1 Gewichsteil Crospovidon sind, wobei der S-Carboxymethylcystein-Gehalt zwischen 40 und 80 Gew.% liegt.

3. Tablette oder Dragéekern nach Anspruch 2, dadurch gekennzeichnet, dass das Hilfsstoffgemisch darin ein Gemisch von zirka 2 Gew.Teile Mannitol, zirka 0,4 Gew.Teile Talk, zirka 0,4 Gew.Teile kolloïdale Kiesesäure, zirka 0,2 Gew.Teile Stearinsäure oder dessen Salz und 1,5 Gew.Teile Stärke auf 1 Gewichtsteil Crospovidon ist.

4. Verfahren zur Herstellung von Tabletten oder Dragéekernen, wobei man S-Carboxymethylcystein mit Mannitol mischt und mit einem Gemisch aus Stärke und Wasser granuliert, trocknet, aufs neue siebt und nach der Hinzufügung der restlichen Hilfstoffe zu Tabletten oder Dragéekernen komprimiert, dadurch gekennzeichnet, dass dabei als Hilfsttofe per Gewichtsteil Crospovidon 1—5 Gew.Teile Mannitol, 0,2—2 Gew.Teile Talk, 0,2—2 Gew.Teile kolloïdale Kieselsäure, 0,1—1 Gew.Teile Stearinsäure oder dessen Salz und 0,2—4 Gew.Teile Stärke verwendet werden, wobei der S-Carboxymethylcystein-Gehalt des Gemisches zwischen 10 und 90 Gew.% liegt.

5. Verfahren nach Anspruch 4, dadurch gekennzeichnet, dass als Hilfsstoffe per Gewichtsteil Crospovidon 1—3 Gew.Teile Mannitol, 0,2—1 Gewichtsteil Talk, 0,2—1 Gew. Teil kolloïdale Kieselsäure, 0,1—0,5 Gew.Teile Stearinsäure oder dessen Salz und 0,5—2 Gew.Teile Stärke verwendet werden.

6. Tablette oder Dragéekern erhältlich mit dem Verfahren nach Anspruch 4 oder 5.

## Revendications

1. Comprimé ou noyau de comprimé contenant de la S-carboxyméthylcystéine et des supports et des adjuvants connus pour des comprimés, caractérisé en ce que les adjuvants dans celui-ci sont un mélange de 1 à 5 parties en poids de mannitol, 0,2 à 2 parties en poids de talc 0,2 à 2 parties en poids de silice colloïdale, 0,1 à 1 partie en poids d'acide stéarique et/ou d'un sel de celui-ci et 0,2 à 4 parties en poids d'amidon pour une partie en poids de Crospovidone, la teneur en S-carboxyméthylcystéine étant de 10 à 90% en poids.

2. Comprimé ou noyau de comprimé suivant la revendication 1, caractérisé en ce que les adjuvants dans celui-ci sont un mélange de 1 à 3 parties en poids de mannitol, 0,2 à 1 partie en poids de talc, 0,2 à 1 partie en poids de silice colloïïdale, 0,1 à 0,5 partie en poids d'acide stéarique et/ou d'un sel de celui-ci et 0,5 à 2 parties en poids d'amidon pour une partie en poids de Crospovidone, la teneur en S-carboxyméthylcystéine étant de 40 à 80% en poids.

3. Comprimé ou noyau de comprimé suivant la revendication 2, caractérisé en ce que les adjuvants dans celui-ci sont un mélange d'environ 2 parties en poids de mannitol, environ 0,4 partie de talc, environ 0,4 partie en poids de silice colloïdale environ 0,2 partie en poids d'acide stéarique ou d'un sel de celui-ci et 1,5 partie en poids d'amidon pour partie en poids de Crospovidone.

4. Procédé pour préparer des comprimés ou des noyaux de comprimés, dans lequel la S-carboxyméthylcystéine est mélangée avec le mannitol et granulée avec un mélange d'amidon et d'eau, séchée, tamisée à nouveau et après addition des adjuvants restants, pressée en comprimés ou en noyaux de comprimées, caractérisé en ce qu'on utilise comme adjuvants pour une partie en poids de Crospovidone, 1 à 5 parties en poids de mannitol, 0,2 à 2 parties en poids de talc, 0,2 à 2 parties en poids de silice colloïdale, 0,1 à 1 partie en poids d'acide stéarique et/ou d'un sel de celui-ci et 0,2 à 4 parties en poids d'amidon, la teneur en S-carboxyméthylcystéine du mélange étant de 10 à 90% en poids.

5. Procédé suivant la revendication 4, caractérisé en ce qu'on utilise comme adjuvants pour une partie en poids de Crospovidone, 1 à 3 parties en poids de mannitol 0,2 à 1 partie en poids de talc, 0,2 à 1 partie en poids de silice colloïde, 0,1 à 0,5 partie en poids d'acide stéarique et/ou d'un sel de celui-ci et 0,5 à 2 parties en poids d'amidon.

6. Comprimé ou noyau de comprimé, caractérisé en ce qu'il peut être obtenu suivant le procédé de la revendication 4 ou 5.